# EUROPÄISCHE PATENTSCHRIFT

(11) **EP 3 166 546 B1**
(45) Veröffentlichungstag und Bekanntmachung des Hinweises auf die Patenterteilung: **31.08.2022**
(21) Anmeldenummer: 15736823.4
(22) Anmeldetag: 09.07.2015
(51) Int. Cl.: A61F 2/64, A61F 2/66, A61F 2/68, A61F 5/01

(54) **ORTHOPÄDIETECHNISCHE GELENKEINRICHTUNG**
ORTHOPEDIC JOINT SYSTEM
DISPOSITIF D'ARTICULATION POUR L'ORTHOPÉDIE

(30) Priorität: 11.07.2014 DE 102014010254
(43) Veröffentlichungstag der Anmeldung: 17.05.2017
(73) Patentinhaber: Ottobock SE & Co. KGaA, 37115 Duderstadt (DE)
(72) Erfinder: BOITEN, Herman, 37085 Göttingen (DE); KIMMIG, Clemens, 77728 Oppenau (DE); DITTRICH, Elmar, 01099 Dresden (DE)
(74) Vertreter: Gramm, Lins & Partner Patent- und Rechtsanwälte PartGmbB
(86) Internationale Anmeldenummer: PCT/EP2015/065684
(87) Internationale Veröffentlichungsnummer: WO 2016/005491

(56) Entgegenhaltungen:
- EP-A2- 2 664 304
- WO-A1-00/74610
- DE-A1- 4 233 247
- DE-U1- 8 515 598
- DE-U1-202004 008 014
- GB-A- 2 194 443
- US-A- 4 034 419
- US-A- 5 888 237

## Beschreibung

Die Erfindung betrifft eine orthopädietechnische Gelenkeinrichtung mit einem Oberteil und einem daran schwenkbar um eine Schwenkachse gelagerten Unterteil vorgesehen, wobei zwischen dem Oberteil und dem Unterteil eine hydraulische Dämpferanordnung eingerichtet ist, die einen in einem Zylinder verdrehsicher gelagerten Kolben mit einer Kolbenstange aufweist.

Orthopädietechnische Gelenkeinrichtungen können insbesondere als sogenannte Sperrkniegelenke ausgebildet sein, die in der Regel in der maximal extendierten Stellung verriegelt werden können, um für den Patienten eine hohe Sicherheit während des Stehens zu gewährleisten. Sperrkniegelenke werden in der Regel für wenig mobile Patienten eingesetzt, bei denen der Fokus darauf gerichtet ist, eine maximale Standsicherheit zu gewährleisten und ein Hinsetzen und Sitzen mit einem abgewinkelten Bein zu ermöglichen. Zum Hinsetzen wird die Sperre gelöst, so dass der Patient sich bei aufgesetztem Fuß hinsetzen kann. Die Hinsetzbewegung kann über eine einfache Dämpfereinrichtung gebremst werden. Ein Prothesenkniegelenk mit einer Sperre und einer hydraulischen Dämpfung zum Hinsetzen ist beispielsweise in der DE 103 51 916 A1 beschrieben.

Die US 4,685,926 A betrifft ein Prothesenkniegelenk mit einer mechanischen Sperre, die über einen Seilzug aufgehoben werden kann. Ein gelenkig gelagerter Hebel ist direkt mit einem Seilzug verbunden und wird bei Betätigung des Seilzuges verschwenkt, so dass ein Sperrzapfen aus einer Ausnehmung heraus bewegt wird.

Die DE 28 48 305 A1 betrifft ein Prothesengelenk zur drehbaren Verbindung zweier Prothesenteile mit einem an einem Prothesenteil drehbar gelagerten und mit dem anderen Prothesenteil fest verbundenen Rastkörper, der an seinem Umfang Rastlücken aufweist, in welche die Nase eines durch den Druck einer Feder gegen den Rastkörper führbaren Riegels einschiebbar ist. Der Riegel dient zur Sperrung des Gelenkes und rastet in die Rastlücken ein. Zur Freigabe des Gelenkes wird der Riegel über einen Seilzug gegen den Druck der Feder herausgezogen. Der Riegel ist, wenn sich die Nase des Riegels innerhalb der Rastlücken des Rastkörpers befindet, über ein zwischen dem Riegel und dem Rastkörper einschiebbares Arretierteil fixierbar.

Die DE 10 2012 009 653 A1 betrifft ein Prothesengelenk mit einem Oberteil, einem Unterteil und einem Sperrelement, wobei das Oberteil und das Unterteil schwenkbar aneinander gelagert sind. Das Sperrelement ist aus einer Freigabestellung in eine Sperrstellung überführbar, wobei in der Sperrstellung das Prothesengelenk in einem vorgebbaren Beugungszustand fixierbar ist und zwischen dem Oberteil und dem Unterteil verspannt wird. Über einen Sperrhebel kann das Sperrelement aus der Sperrstellung in die Freigabestellung überführt werden. Das Sperrelement ist im Oberteil gelagert, wobei das Oberteil eine Aussparung aufweist, in der das Sperrelement verschiebbar gelagert ist.

Die DE 20 2008 002 764 U1 betrifft einen Entriegelungsmechanismus für gesperrte Kniegelenke in der Orthopädietechnik, bei dem durch Fingerdruck auf einen Stößel ein Hebel aus seiner Ausgangslage in eine Endlage bewegt wird. Dabei erfolgt eine Bewegungsumlenkung von 90° und eine Übersetzung im Verhältnis 1:2, so dass aus einer horizontalen Stößelbewegung eine vertikale Bewegung des Hebels um den doppelten Weg wird. Durch die Betätigung erfolgt eine Entriegelung gegen eine Feder im Kniegelenk, die eine Rückstellkraft in die Ausgangslage bewirkt. Der Entriegelungsmechanismus ist eingegossen.

Die DE 85 15 598 U1 betrifft ein arretierbares Kniegelenk mit einem Oberschenkelteil, einem daran schwenkbar gelagerten Unterschenkelteil und einer Arretiervorrichtung, bei der ein längsverlagerbarer Verriegelungsbolzen innerhalb eines Montageblockes in eine Ausnehmung auf der Unterseite des Oberschenkelteils hineinragt. Der Verriegelungsbolzen ist über einen zweiarmigen Hebel mit einem Bowdenzug verbunden und über ein Auslöseteil betätigbar. Wird ein Hebel an dem Auslöseteil betätigt, verkürzt sich der Abstand zwischen einem Wiederlager des Bowdenzuges und einem Wippenarm, so dass der Verriegelungsbolzen aus der Ausnehmung herausgezogen wird.

Die DE 42 33 247 A1 wird als nächstliegender Stand der Technik angesehen und betrifft eine Schwungphasensteuervorrichtung für ein künstliches Prothesenkniegelenk mit einem Hydraulikdämpfer, der eine Kolben-Zylinder-Einrichtung aufweist. Der Kolben ist längsverschieblich in einem Zylinder an einer Kolbenstange geführt. Die beiden durch den Kolben getrennten Kammern sind durch eine einstellbare erste Drossel miteinander verbunden. Innerhalb der Kolbenstange ist eine Steuerstange geführt, die ein Rändelteil aufweist und über ein Gewinde mit der Kolbenstange verbunden ist. An einem Ende weist die Steuerstange einen konischen Abschnitt auf, der mit einem konischen Abschnitt einer konzentrischen Bohrung innerhalb des Kolbens zusammenwirkt. Durch Drehen an dem Rändelteil kann die Steuerstange in den konischen Abschnitt bis zu einer Nullstellung hineingedreht oder aus ihm herausgedreht werden, so dass eine einstellbare Drossel gebildet ist. Die DE 20 2004 008 014 U1 betrifft eine Schwungphasensteuervorrichtung für ein künstliches Prothesenkniegelenk mit einem Hydraulikdämpfer, der eine Kolben-Zylinder-Einrichtung aufweist. Der Kolben ist längsverschieblich in einem Zylinder an einer Kolbenstange geführt. Die beiden durch den Kolben getrennten Kammern sind durch einstellbare Drosseln miteinander verbunden. Bei Erreichen eines vorbestimmten Druckes werden die Drosseln geschlossen. Eine Verstellung wird über eine Steuerstange oder ein Steuerelement durchgeführt. Eine gewisse Verdrehsicherheit wird über eine entsprechende Lagerung der Kolbenstange bzw. des Zylinders über Bolzen an dem Oberteil oder Unterteil erreicht.

Aufgabe der vorliegenden Erfindung ist es, eine orthopädietechnische Gelenkeinrichtung bereitzustellen, das einen möglichst kleinen Bauraum benötigt und preiswert aufgebaut ist, um die Kosten für eine solche orthopädietechnische Gelenkeinrichtung zu minimieren.

Erfindungsgemäß wird diese Aufgabe durch eine orthopädietechnische Gelenkeinrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Eine nicht von der Erfindung erfasste orthopädietechnische Gelenkeinrichtung mit einem Oberteil und einem daran verschwenkbar um eine Schwenkachse gelagerten Unterteil und einer Sperrklinke, die in einer Verriegelungsstellung eine Relativbewegung von Oberteil zu Unterteil um die Schwenkachse sperrt und in einer Freigabestellung eine Relativbewegung um die Schwenkachse zulässt, wobei in der Verriegelungsstellung die Sperrklinke formschlüssig in ein Sperrelement eingreift und über ein Zugelement in die Freigabestellung verlagerbar ist, wobei das Zugelement mit einem ersten Ende an dem Oberteil oder Unterteil festgelegt und zumindest in der Verriegelungsstellung der Sperrklinke in einem Bogenabschnitt außer Eingriff außen an der Sperrklinge entlanggeführt und ein zweites Ende des Zugelementes mit einer Betätigungseinrichtung versehen ist, sieht vor, dass durch Betätigen der Betätigungseinrichtung der Bogenabschnitt verkürzt wird und die Sperrklinke in die Freigabestellung bringbar ist. Durch die Vorbeiführung des Bogenabschnittes an der Sperrklinke ist es möglich, auf eine Hebelanordnung mit einem zweiarmigen Hebel zu verzichten, da das Zugelement nicht mehr dazu eingesetzt wird, einen Hebel zu verschwenken, um eine Sperrklinke oder einen Zapfen außer Eingriff mit einem Sperrelement zu bringen. Vielmehr wird durch die Verkürzung des Zugelementes im Bereich des Bogenabschnittes die Sperrklinke in die Freigabestellung bewegt. Der Bogenabschnitt des Zugelementes, der außen an der Sperrklinke oder einem daran befestigten oder daran ausgebildeten Anlageelement geführt ist, bewirkt eine direkte Krafteinleitung, so dass auf Umlenkhebel, eine zweiarmige Hebellösung sowie Umlenkeinrichtungen für das Zugelement verzichtet werden kann. Der Bogenabschnitt wird begradigt, das Zugelement steht in direktem Kontakt mit der Sperrklinke, so dass ohne eine Hebellösung oder Umlenkelemente durch die Verkürzung des Bogenabschnittes beim Ziehen des Zugelementes die Sperrklinke außer Eingriff mit dem Sperrelement gebracht wird und die orthopädietechnische Gelenkeinrichtung entriegelt. Das Zugelement kann als Seil, Draht oder Band ausgebildet sein und ist bevorzugt elastisch aber zugstabil, das heißt, dass es sich durch Aufbringen einer Zugkraft nicht oder nur unwesentlich längt.

Die orthopädietechnische Gelenkeinrichtung kann als Gelenkeinrichtung sowohl in einer Orthese als auch in einer Prothese eingesetzt werden und als Gelenkeinrichtung für ein Knie-, Knöchel-, Hüft-, Schulter-, Ellbogen-, Hand- oder Fingergelenk ausgebildet und ausgeführt sein.

Das Sperrelement, in das die Sperrklinke formschlüssig eingreift, kann als eine Ausnehmung in dem Oberteil oder in dem Unterteil ausgebildet sein, alternativ kann an dem Unterteil oder dem Oberteil ein Vorsprung ausgebildet oder angeordnet sein, so dass die Sperrklinke so den Vorsprung hintergreift, dass eine formschlüssige Verriegelung zwischen der Sperrklinke und dem Sperrelement vorliegt. Die Ausgestaltung als Ausnehmung erleichtert die Führung des Zugelementes im Bereich der Ausnehmung, vorteilhafterweise innerhalb der Ausnehmung.

Vorteilhafterweise ist das erste Ende des Zugelementes nicht an der Sperrklinke befestigt, sondern nur an dem Oberteil bzw. Unterteil, wobei vorteilhafterweise die Festlegung des ersten Endes an derjenigen Komponente erfolgt, an der auch die Sperrklinke gelagert ist, also entweder an dem Oberteil, wenn an dem Oberteil die Sperrklinke verdrehbar oder verschieblich gelagert ist oder umgekehrt an dem Unterteil, wobei das Sperrelement an dem korrespondierenden anderen Teil der orthopädietechnischen Gelenkeinrichtung angeordnet, ausgebildet oder befestigt ist.

Das Zugelement kann an einer Sperrklinkennase oder an einem an der Sperrklinke angeordneten Anlegeelement geführt sein. Die Führung des Zugelementes an der Sperrklinkennase hat den Vorteil, dass das Formschlusselement der Sperrklinke gleichzeitig als Anlage für das Zugelement dient, so dass keine weiteren Komponenten oder Bauteile mehr benötigt werden, um ein Zugelement zu führen. Das Vorsehen eines Anlageelementes an der Sperrklinke vergrößert die Möglichkeiten, das Zugelement so zu führen, dass ein Nutzer es einfach bedienen kann bzw. eine verbesserte Führung des Zugelementes erreicht werden kann, so dass die Betätigungsfunktion zuverlässig und einfach ausführbar ist.

Die Sperrklinke kann in Richtung der Verriegelungsstellung elastisch vorgespannt sein, beispielsweise durch eine Feder oder ein Elastomerelement, so dass nach dem Entriegeln des Prothesenkniegelenkes und einem Verschwenken des Oberteils relativ zu dem Unterteil aus der gestreckten Stellung in eine gebeugte Stellung nach dem Aufstehen oder Strecken des Kniegelenkes automatisch eine Verriegelung der Sperrklinke in dem Sperrelement oder mit dem Sperrelement erfolgt. Dadurch wird sichergestellt, dass nach Erreichen der Extensionsstellung die orthopädietechnische Gelenkeinrichtung, z.B. das Prothesenkniegelenk, Orthesenkniegelenk oder ein anderes Prothesen- oder Orthesengelenk, verriegelt ist und dem Patienten eine maximale Standsicherheit oder Stabilität zur Verfügung gestellt wird.

Das Zugelement kann zwischen der Sperrklinke und dem Sperrelement geführt sein, also z.B. zwischen der Sperrklinke und dem Sperrelement eingeklemmt sein, so dass es innerhalb der Formschlussanordnung von Sperrklinke und Sperrelement positioniert ist. Das Zugelement kann in einer speziell ausgebildeten Führung in dem Sperrelement oder der Sperrklinke geführt sein, beispielsweise kann in dem Sperrelement, wenn dieses als Ausnehmung ausgebildet ist, eine Nut oder ein Schlitz eingearbeitet sein, in dem das Zugelement geführt ist und in der Verriegelungsstellung eingelegt ist. Der Bogenabschnitt wird dann in der Führung ausgebildet.

Das erste Ende des Zugelementes kann an derjenigen Komponente des Prothesenkniegelenkes festgelegt sein, die das Sperrelement aufweist, wobei diese Komponente gleichzeitig auch die Zugmittelführung aufweist, beispielsweise den Schlitz oder die Nut, in dem das Zugelement eingelegt ist, so dass zwar eine Blockierung durch die Sperrklinke erfolgt, ein direktes Einklemmen und Quetschen des Zugelementes jedoch vermieden wird.

Erfindungsgemäß ist eine orthopädietechnische Gelenkeinrichtung gemäß Anspruch 1 mit einem Oberteil und einem daran schwenkbar um eine Schwenkachse gelagerten Unterteil vorgesehen, wobei zwischen dem Oberteil und dem Unterteil eine hydraulische Dämpferanordnung eingerichtet ist, die einen in einem Zylinder verdrehsicher gelagerten Kolben mit einer Kolbenstange aufweist, wobei in dem Kolben ein über die Kolbenstange verstellbarer Einsatz mit einem veränderlichen Drosselspalt angeordnet ist. Durch die zusätzliche Anordnung einer Dämpfereinrichtung kann dem Patienten eine Hinsetzhilfe oder eine Dämpfung der Rotationsbewegung zur Verfügung gestellt werden, da der Dämpfer eine Lastübernahme bewirkt, so dass sich der Patient nicht vollständig mit seinem Gewicht auf dem unversorgten Bein oder über die oberen Extremitäten an Gehhilfen oder anderen Gegenständen abstützen oder bei der Anordnung der Gelenkeinrichtung an anderer Stelle die vollständige Last, die auf das Gelenk einwirkt, aufnehmen muss.

Durch den veränderlichen Drosselspalt ist es möglich, die Dämpfungsrate des hydraulischen Dämpfers individuell einzustellen. Die Einstellbarkeit über die Kolbenstange erleichtert aufgrund der leichten Zugänglichkeit der Kolbenstange die Einstellprozedur.

Der verstellbare Einsatz kann in Verlagerungsrichtung des Kolbens verschieblich oder rund und innerhalb des Kolbens verdrehbar gelagert sein, um durch die Ortsveränderung des Einsatzes innerhalb des Kolbens den Querschnitt des Drosselspaltes zu verändern, zur Erhöhung der Dämpfung den Drosselspalt zu verkleinern und zur Verringerung der Dämpfung diesen zu vergrößern.

Der Einsatz kann in einem verdrehgesichert in dem Kolben gelagerten Einstellring gelagert sein, so dass beispielsweise Kanäle und/oder umlaufende oder teilweise umlaufende Nuten zur Ausbildung eines Drosselspaltes einfach in dem Einstellring oder dem Einsatz gefertigt und anschließend in dem Kolben endmontiert werden können. Der Kolben kann aus dem Einsatz und dem Einstellring bestehen und die Trennung zwischen einer Extensionskammer und einer Flexionskammer bewirken.

An dem Einstellring und in dem Einsatz können jeweils ein Kanal ausgebildet sein, der mit dem Drosselspalt strömungstechnisch verbunden ist, wobei der Drosselspalt zwischen dem Einstellring und dem Einsatz ausgebildet ist. Über den Kanal wird die jeweilige Verbindung von der Flexionskammer zu dem Drosselspalt bzw. der Extensionskammer zu dem Drosselspalt hergestellt.

Zur Veränderung des Querschnitts des Drosselspaltes kann dieser einen über den Verstellweg des Einsatzes veränderlichen Querschnitt aufweisen, beispielsweise indem die Tiefe einer Nut, die bei einem runden Einsatz den Drosselspalt in Verbindung mit dem Einstellring ausbildet, über den Umfang vergrößert wird, so dass bei einer entsprechenden Verdrehung eine verringerte Menge des Hydraulikfluids durch den Drosselspalt hindurch gelangen kann.

Der Kolben und der Zylinder sind vorteilhafterweise oval ausgebildet, um allein durch die Formgebung des Kolbens eine Verdrehsicherheit innerhalb des Zylinders zu bewirken. Die ovale Formgebung ermöglicht einen schmalen Aufbau des Hydraulikdämpfers bei gleichzeitig gegebener Abdichtbarkeit und einfacher Fertigung.

Vorteilhafterweise sind sowohl der Kolben als auch der Zylinder aus einem Kunststoff gefertigt, um den Hydraulikdämpfer möglichst leicht bauen zu können. Eine Variante der Erfindung sieht vor, dass entweder der Kolben oder der Zylinder vollständig aus einem Kunststoff ausgebildet sind, um einerseits eine einfache Fertigung und ein geringes Gewicht und andererseits eine hohe Belastbarkeit durch die Wahl eines anderen Werkstoffes zu erreichen. Der Kolben kann aus einem Metall, beispielsweise Stahl oder Aluminium gefertigt sein und einen Kolbenring aus Kunststoff aufweisen, insbesondere wenn der Zylinder aus einem Kunststoff gefertigt ist. Dies ermöglicht eine leichte Bauweise des Hydraulikdämpfers. Der Kolben kann auch nur teilweise aus einem Metall bestehen, z.B. der Einstellring, an dessen Umfang ein Kolbenring aus Kunststoff in einer Ringnut festgelegt ist und in dem der Einsatz, der aus Kunstsoff gefertigt sein kann, eingesetzt ist. Umgekehrt kann der Einsatz mit der Verbindung zur Kolbenstange aus einem Metall ausgebildet und in einem Einstellring aus Kunststoff gelagert sein. Ist der Zylinder aus einem Metall gefertigt, ist es Vorteilhaft, den Kolbenring ebenfalls aus einem Metall zu fertigen, allerdings nicht unbedingt notwendig.

In dem Kolben ist zumindest ein Rückschlagventil angeordnet, das einem Bypass des Drosselspaltes verschließt, so dass eine Extension des Prothesenkniegelenkes bei entsprechender Ausrichtung des Rückschlagventils stets ungehindert oder nahezu ungehindert möglich ist.

Ein Rückschlagventil kann auch mit einer Vorspannkraft federbelastet sein, so dass erst ab Erreichen eines bestimmten Druckes innerhalb der Flexionskammer oder Extensionskammer das Ventil öffnet und die Kurzschlussleitung zwischen der Extensionskammer und der Flexionskammer in Gestalt des Bypasses des Drosselspaltes freigibt.

Nachfolgend werden Ausführungsbeispiele der Erfindung anhand der beigefügten Figuren näher erläutert. Es zeigen:
- Figur 1: eine perspektivische Gesamtansicht eines Prothesenkniegelenkes;
- Figur 2: eine Schnittdarstellung gemäß Figur 1;
- Figur 3a: eine schematische Darstellung des Prothesenkniegelenkes in Verriegelungsstellung;
- Figur 3b: eine Anordnung gemäß Figur 3a in Freigabestellung;
- Figur 3c: eine Draufsicht auf eine Sperrklinke;
- Figur 4: eine Detaillängsschnittdarstellung des Hydraulikdämpfers; sowie
- Figur 5: eine Querschnittsansicht des Hydraulikdämpfers.

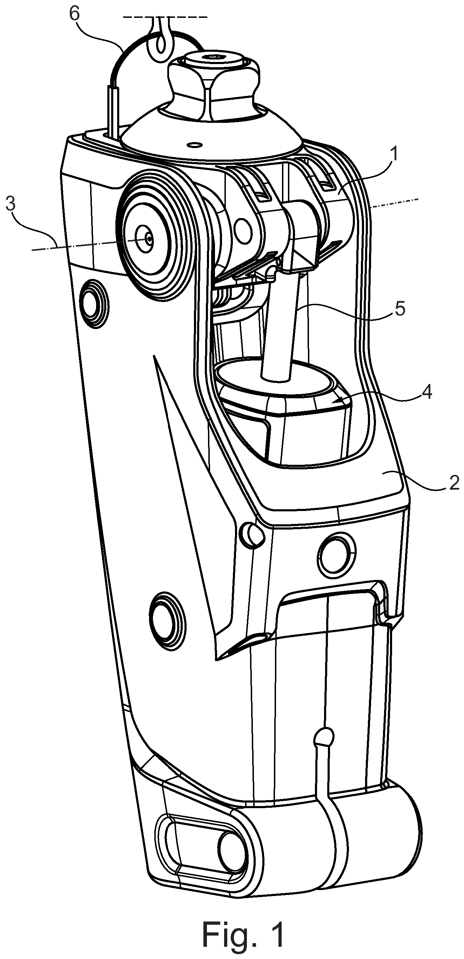

Die Figur 1 zeigt in einer perspektivischen Ansicht ein Prothesenkniegelenk in Gestalt eines Sperrkniegelenkes mit einem Oberteil 1, das an einem Unterteil 2 um eine Schwenkachse 3 gelagert ist. Das Prothesenkniegelenk ist als 1-Achs-Kniegelenk ausgebildet, dessen Oberteil 1 in Gehrichtung hinter der Schwenkachse 3 eine Lagerachse für eine Kolbenstange 5 eines Hydraulikdämpfers 4 aufweist. Der Hydraulikdämpfer 4 dämpft die Verschwenkbewegung des Oberteils 1 relativ zu dem Unterteil 2 um die Schwenkachse aus der dargestellten geraden, extendierten und verriegelten Stellung in eine flektiert, eingebeugte Stellung, beispielsweise beim Sitzen oder Hinsetzen. Das Prothesenkniegelenk wird durch eine in der Figur 1 nicht gezeigte Sperrklinke formschlüssig verriegelt, die Verriegelung kann über ein Zugelement 6 gelöst werden.

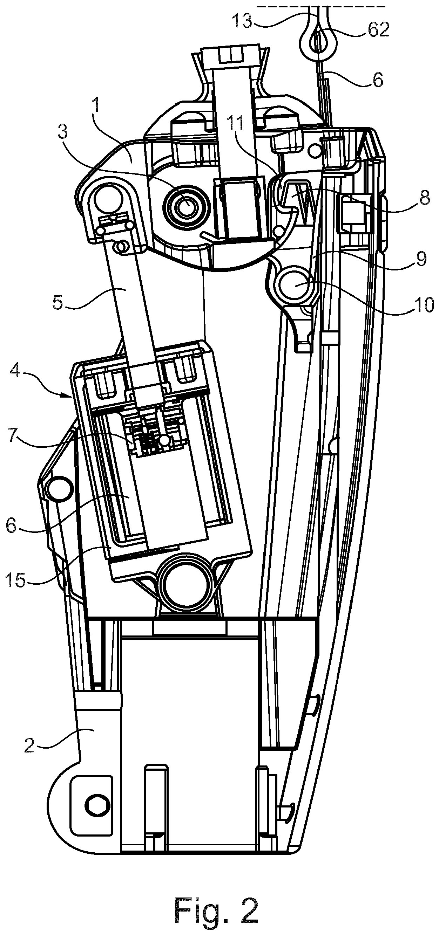

Figur 2 zeigt das Prothesenkniegelenk der Figur 1 in einer Schnittansicht. Das Oberteil 1 ist an dem Unterteil 2 um die senkrecht zur Zeichenebene ausgerichtete Schwenkachse 3 verschwenkbar gelagert. Der Hydraulikdämpfer 4 ist sowohl an dem Oberteil 1 als auch an dem Unterteil 2 über Achsen verschwenkbar gelagert. Der Hydraulikdämpfer 4 ist mit dem Oberteil 1 über eine Kolbenstange 5 verbunden, an dem Unterteil 2 ist das Dämpfergehäuse 15 gelagert. Innerhalb des Dämpfergehäuses 15 ist ein ovaler Zylinder 6 angeordnet, in dem ein Kolben 7, der mit der Kolbenstange 5 gekoppelt ist, längsverschieblich gelagert ist. Der Kolben 7 trennt eine Extensionskammer des Hydraulikzylinders 4 strömungstechnisch von einer Flexionskammer, wobei eine Überströmeinrichtung in Gestalt eines Drosselspaltes, der näher später erläutert wird, vorgesehen ist.

An dem Unterteil 2 ist ebenfalls verschwenkbar eine Sperrklinke 8 angeordnet, die um eine Schwenkachse 10 beweglich gelagert ist. Über eine Feder 9 wird die Sperrklinke 8 in Richtung auf ein Sperrelement 11 vorgespannt. Das Sperrelement 11 ist in Gestalt einer Ausnehmung ausgebildet, in die die Sperrklinke 8, die ein Formschlusselement in Gestalt einer Sperrnase aufweist formschlüssig eingreift. Die Ausnehmung 11 und die Sperrnase der Sperrklinke 8 sind korrespondierend geformt, so dass in dem dargestellten verriegelten Zustand eine Verschwenkung des Oberteils 1 relativ zu dem Unterteil 2 um die Schwenkachse 3 verhindert wird.

Der Sperrklinke 8 ist ein Zugelement 6 zugeordnet, das an der Sperrklinke 8 entlang geführt ist und mit einem nicht dargestellten ersten Ende an dem Oberteil 1 oder dem Unterteil 2 festgelegt ist. Das Zugelement 6 ist in der dargestellten Verriegelungsstellung in einem Bogen entlang der Sperrklinke 8 geführt und mit einem zweiten Ende 62, das mit einer Betätigungseinrichtung 13 gekoppelt ist, versehen. Wird die Betätigungseinrichtung 13 nach oben gezogen oder das zweite Ende 62 unmittelbar in Proximalrichtung verlagert, verkürzt sich der Bogen, die Sperrklinke 8 wird um die Schwenkachse 10 entgegen der Feder 9 außer Eingriff mit der Ausnehmung 11 gebracht und eine Freigabestellung erreicht.

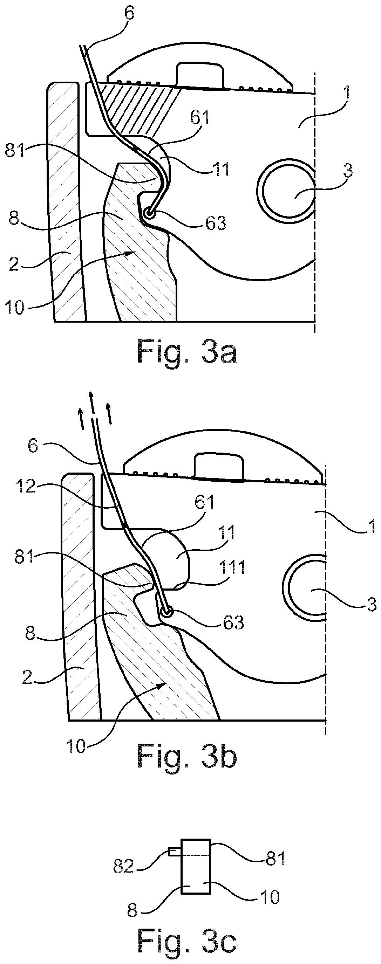

Figur 3a zeigt die Anordnung des Zugelements 6, der Sperrklinke 8 und des Oberteils 1 in schematischer Darstellung in Verriegelungsstellung. Ein erstes Ende 63 ist an dem Oberteil 1 festgelegt, der Bogenabschnitt 61 des Zugelementes 6 ist an dem vorderen Ende der Verrieglungsnase 81 der Sperrklinke 8 geführt, so dass bei einem nicht betätigten Zugelement 6 ein lockeres Anliegen des Bogenabschnittes 61 außen an der Sperrklinkennase 81 vorhanden ist. Der Bogenabschnitt 61 ist nicht an der Sperrklinke 8 befestigt, sondern liegt nur an der Außenseite an. Die Sperrklinke 8 ist um die Schwenkachse 10 verschwenkbar an dem Unterteil 2 gelagert. In der dargestellten Verriegelungsstellung ist die Verdrehung des Oberteils 1 gegenüber dem Unterteil 2 um die Schwenkachse 3 gesperrt.

Figur 3b zeigt die Funktionsweise der Entriegelung. Das Zugelement 6 wird in Pfeilrichtung nach oben gezogen, dadurch streckt sich das Zugelement 6 und hat die Tendenz, den Bogenabschnitt 61 zu begradigen. Dadurch drückt das Zugelement 6 von außen auf den vorderen Abschnitt der Sperrklinkennase 81, so dass die Sperrklinke 8 um die Schwenkachse 10 entgegen der Vorspannkraft der nicht dargestellten Feder außer Eingriff mit der Ausnehmung 11 innerhalb des Oberteils 1 gebracht wird. Die formschlüssige Verriegelung durch die Sperrklinkennase 81 ist aufgehoben, die Sperrklinkennase 81 ist von der korrespondierenden Anlagefläche 111 in der Ausnehmung 11 entfernt und das Zugelement 6 wird in eine gestreckte Stellung, ausgehend von dem Festlegungspunkt des ersten Zugelementelendes 63 gebracht. Innerhalb des Oberteils 1 ist eine Zugelementführung 12 in Gestalt eines Schlitzes und einer Führungsschräge ausgebildet, so dass das Zugelement 6 sich nicht aus der Ausnehmung 11 heraus bewegen kann. Die Führung 12 des Zugelementes 6 kann auch als Kanal mit drei Anlageseiten oder auch als eine geschlossene Durchführung in Gestalt einer Bohrung ausgebildet sein.

Figur 3c zeigt in einer schematischen Darstellung eine Sperrklinke in einer Draufsicht. Die Verschwenkbarkeit um die Schwenkachse 10 ist angedeutet, neben einer Ausgestaltung der formschlüssigen Verriegelung über eine Sperrnase 81 ist es möglich, seitlich neben der Sperrnase 81 ein Anlageelement 82 anzubringen oder vorzusehen, um dort das Zugelement 6 zu führen.

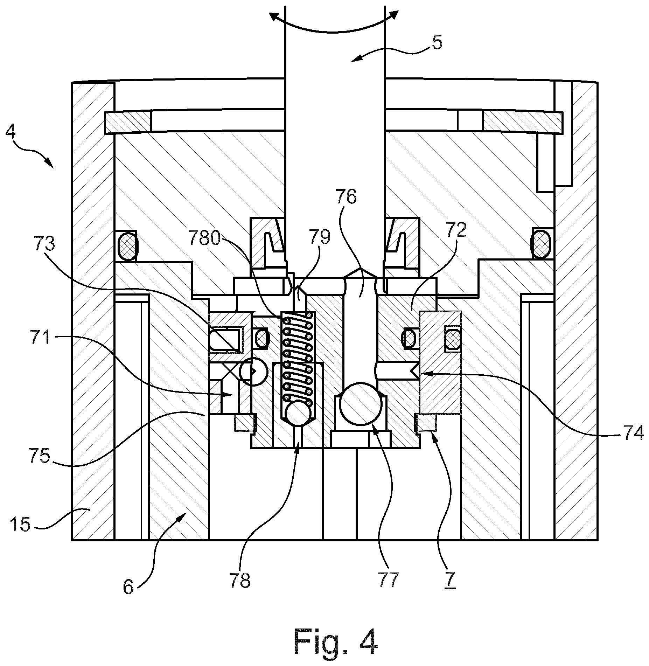

Figur 4 zeigt eine Schnittdarstellung durch einen Teil des Hydraulikdämpfers 4. Der Hydraulikdämpfer 4 sieht in seinem Gehäuse 15 einen ovalen Zylinder 6 vor, in dem ein Kolben 7 angeordnet ist, der eine Extensionskammer von einer Flexionskammer trennt. Der Kolben 7 ist über eine Kolbenstange 5 mit dem Oberteil 1 des nicht dargestellten Prothesenkniegelenks verbunden. Der Kolben 7 besteht aus einem außen ovalen Einstellring 71, der in Längserstreckung der Kolbenstange 5 verschieblich und verdrehgesichert innerhalb des Zylinders 6 gelagert ist. Zentral innerhalb des Einstellrings 71 ist ein runder Einsatz 72 verdrehbar angeordnet, wobei der Einsatz 72 drehfest mit der Kolbenstange 5 gekoppelt ist. Die Verdrehbarkeit der Kolbenstange 5 ist durch den Doppelpfeil angedeutet. Wird nun ein Druck durch Einbeugen des Prothesenkniegelenkes auf die Kolbenstange 5 ausgeübt, strömt Hydraulikfluid von der unteren Flexionskammer durch einen Kanal 75 in den Einstellring 71 zu einem Drosselspalt 73, der zwischen dem Einstellring 71 und dem Einsatz 72 ausgebildet ist. Der Drosselspalt 73 wird durch eine umlaufende oder teilumlaufende Nut innerhalb des Einsatzes 72 ausgebildet, wobei die Dämpfungswirkung des Hydraulikdämpfers durch den geringsten Querschnitt definiert wird, die im dargestellten Ausführungsbeispiel an dem eigekreisten Drosselspalt 73 befindlich ist. Um den Umfang des Einsatzes 72 herum ist eine sich in Umfangsrichtung vertiefende Nut ausgebildet, die strömungstechnisch in Verbindung mit einem zweiten Kanal 76 steht, so dass bei Flexion des Prothesenkniegelenkes 1 das Hydraulikfluid aus der Extensionskammer durch den Kanal 75, durch den Drosselspalt 73 und durch den zweiten Kanal 76 in die Flexionskammer einströmt. Der Kanal 76 steht in unmittelbarer strömungstechnischer Verbindung mit der Flexionskammer.

Innerhalb des Kanals 76 ist ein Rückschlagventil 77 angeordnet, das einen direkten Durchgang von der Extensionskammer zur Flexionskammer bei Flexion des Prothesenkniegelenkes verhindert. Bei einer Bewegungsumkehr, also bei einer Extension des Unterteils 2 relativ zum Oberteil 1, strömt das Hydraulikfluid von der Flexionskammer durch den Kanal 76 in Richtung auf das Rückschlagventil 77, verschiebt die Sperrkugel aus dem Ventilsitz und ermöglicht ein Rückströmen des Fluids von der Flexionskammer in die Extensionskammer in nahezu ungehinderter Weise. Dadurch ist es möglich, einen definierten Widerstand gegen eine Flexion durch die Variation des Querschnittes des Drosselspaltes 73 zu erreichen, ohne das Aufstehen wesentlich zu behindern.

Zusätzlich ist in dem Einsatz 72 ein weiterer Kanal 79 vorgesehen, der durch ein Rückschlagventil 78, das über eine Feder 780 in den Ventilsitz gepresst wird, verschlossen ist. Das zweite Rückschlagventil 78 wirkt dem ersten Rückschlagventil 77 entgegengesetzt und dient als Überlastventil, das eine mögliche mechanische Beschädigung aufgrund zu hoher Drücke verhindert oder bei einer falschen Einstellung des Drosselspaltquerschnittes eine Beschädigung und ein Einbeugen des Prothesenkniegelenkes auch im Notfall ermöglicht.

Statt der dargestellten rotatorischen Einstellbarkeit des Querschnittes des Drosselspaltes 73 ist es möglich, durch Verdrehung der Kolbenstange 5 relativ zu dem drehfest gelagerten Kolben 7 eine Axialverschieblichkeit des Einsatzes 72 zu bewirken, um durch eine entsprechende Relativverlagerung zu dem Einstellring 71 eine Veränderung des Drosselspaltquerschnittes zu ermöglichen.

Die Ausgestaltung des Hydraulikdämpfers 4 in der dargestellten Form mit der Konstruktion mit dem drehfesten jedoch axial verschieblichen Kolben 7 ist auch ohne das Prothesenkniegelenk als einfache Hydraulikdämpfung mit einer leichten Einstellbarkeit und einer preiswerten Herstellung aufgrund der Verwendung von Kunststoffen eine eigenständige Lösung der Aufgabe.

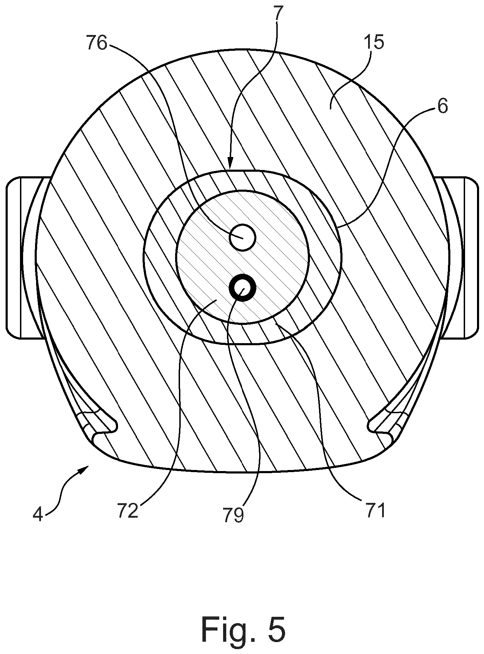

In der Figur 5 ist eine Schnittdarstellung durch den Hydraulikdämpfer 4 auf Höhe des Kolbens 7 senkrecht zu dessen Verlagerungsrichtung gezeigt. Der Zylinder 6 ist in dem Gehäuse 15 ausgebildet und weist eine ovale Kontur auf. In dem Zylinder 6 ist der Kolben 7 angeordnet, der ebenfalls eine korrespondierende, ovale Außenkontur aufweist. Der Kolben 7 weist einen äußeren Einstellring 71 als Kolbenbestandteil auf, der die ovale und dadurch verdrehsichere Außenkontur ausbildet und eine runde Innenausnehmung aufweist, in der verdrehbar der Einsatz 72 gelagert ist. In dem Einsatz 72 sind die beiden Kanäle 76, 79 zu erkennen, die eine Verbindung zwischen der Extensionskammer und der Flexionskammer ermöglichen. Der obere Kanal 76 steht mit der den Drosselspalt 73 ausbildenden Nut über eine radiale Bohrung in Verbindung, so dass bei einem geöffneten Drosselspalt eine strömungstechnische Verbindung von der Flexionskammer durch den Kanal 75 in der Figur 4, den Drosselspalt 73 und die radiale Bohrung und den Kanal 76 zur Extensionskammer bewirkt wird. Der untere Kanal 79 dient als strömungstechnische Verbindung und Bypass bei einer Überbelastung, so dass bei einem zu hohen Druck innerhalb der Flexionskammer das als Rückschlagventil ausgebildete Sicherheitsventil öffnet und somit ein Einbeugen der Gelenkeinrichtung ermöglicht. In dem ovalen Kolben 7 ist der in dem Einstellring 71 verdrehbare Einsatz 72 gelagert, der über eine Verdrehung der Kolbenstange 5 um ihre Längsachse in seiner Position oder Orientierung in dem Einstellring 71 einstellbar ist, so dass der Strömungswiderstand durch eine Veränderung des Querschnitts des Drosselspaltes verändert werden kann.

## Patentansprüche

1. Orthopädietechnische Gelenkeinrichtung mit einem Oberteil (1) und einem daran verschwenkbar um eine Schwenkachse (3) gelagerten Unterteil (2), wobei zwischen dem Oberteil (1) und dem Unterteil (2) eine hydraulische Dämpfereinrichtung (4) angeordnet ist, die einen in einem Zylinder (6) gelagerten Kolben (7) mit einer Kolbenstange (5) aufweist, **dadurch gekennzeichnet, dass** der Kolben (7) verdrehsicher in dem Zylinder (6) gelagert ist, und in dem Kolben (7) ein über die Kolbenstange (5) verstellbarer Einsatz (72) mit einem veränderlichen Drosselspalt (73) angeordnet ist.

2. Orthopädietechnische Gelenkeinrichtung nach Anspruch 1, **dadurch gekennzeichnet, dass** der Einsatz (72) in Verlagerungsrichtung des Kolbens (7) verschieblich oder rund ausgebildet und verdrehbar in dem Kolben (7) gelagert ist.

3. Orthopädietechnische Gelenkeinrichtung nach Anspruch 1 oder 2, **dadurch gekennzeichnet, dass** der Einsatz (72) in einem verdrehgesichert in dem Kolben (7) gelagerten Einstellring (71) gelagert ist.

4. Orthopädietechnische Gelenkeinrichtung nach Anspruch 3, **dadurch gekennzeichnet, dass** in dem Einstellring (71) und in dem Einsatz (72) jeweils ein Kanal (75, 76) ausgebildet ist, der mit dem Drosselspalt (73), der zwischen dem Einstellring (71) und Einsatz (72) ausgebildet ist, strömungstechnisch verbunden.

5. Orthopädietechnische Gelenkeinrichtung nach einem der Ansprüche 1 bis 4, **dadurch gekennzeichnet, dass** der Drosselspalt (73) einen über einen Verstellweg des Einsatzes (72) veränderlichen Querschnitt aufweist.

6. Orthopädietechnische Gelenkeinrichtung nach einem der Ansprüche 1 bis 5, **dadurch gekennzeichnet, dass** der Kolben (6) und Zylinder (7) oval ausgebildet sind.

7. Orthopädietechnische Gelenkeinrichtung nach einem der Ansprüche 1 bis 6, **dadurch gekennzeichnet, dass** in dem Kolben (7) zumindest ein Rückschlagventil (77, 78) angeordnet ist, das einen Bypass (76, 79) des Drosselspaltes (73) verschließt.

8. Orthopädietechnische Gelenkeinrichtung nach Anspruch 7, **dadurch gekennzeichnet, dass** zumindest ein Rückschlagventil (77, 78) federbelastet ist.

9. Orthopädietechnische Gelenkeinrichtung nach einem der Ansprüche 1 bis 8, **dadurch gekennzeichnet, dass** der Kolben (7) und/oder der Zylinder (6) zumindest teilweise aus einem Kunststoff gefertigt sind.

10. Orthopädietechnische Gelenkeinrichtung nach einem der voranstehenden Ansprüche, **dadurch gekennzeichnet, dass** die Gelenkeinrichtung als Sperrkniegelenk ausgebildet ist.

## Claims

1. An orthopedic joint system having an upper part (1) and a lower part (2), which is mounted on the upper part such that it can be pivoted about a pivot pin (3), wherein the upper part (1) and the lower part (2) have arranged between them a hydraulic damper device (4), which has a piston (7) which is mounted in a cylinder (6) and has a piston rod (5), **characterized in that** the piston (7) is mounted in a rotationally secured manner in the cylinder (6) and that an insert (72), which can be adjusted via the piston rod (5) and has a variable throttle gap (73), is arranged in the piston (7).

2. The orthopedic joint system as claimed in claim 1, **characterized in that** the insert (72) is designed to be round or displaceable in the direction in which the piston (7) is shifted, and it is mounted in a rotatable manner in the piston (7).

3. The orthopedic joint system as claimed in claim 1 or 2, **characterized in that** the insert (72) is mounted in an adjustment ring (71), which is mounted in a rotationally secured manner in the piston (7).

4. The orthopedic joint system as claimed in claim 2, **characterized in that** the adjustment ring (71) and the insert (72) each respectively contain a channel (75, 76) which is connected in flow terms to the throttle gap (73), which is formed between the adjustment ring (71) and insert (72) .

5. The orthopedic joint system as claimed in one of claims 1 to 4, **characterized in that** the throttle gap (73) has a cross section which is variable over an adjustment path of the insert (72).

6. The orthopedic joint system as claimed in one of claims 1 to 5, **characterized in that** the piston (6) and cylinder (7) are of oval design.

7. The orthopedic joint system as claimed in one of claims 1 to 6, **characterized in that** the piston (7) contains at least one nonreturn valve (77, 78), which closes a bypass (76, 79) of the throttle gap (73).

8. The orthopedic joint system as claimed in claim 7, **characterized in that** at least one nonreturn valve (77, 78) is spring-loaded.

9. The orthopedic joint system as claimed in one of claims 1 to 8, **characterized in that** the piston (7) and/or the cylinder (6) are/is produced, at least in part, from a plastics material.

10. The orthopedic joint system as claimed in one of the preceding claims, **characterized in that** the joint system is designed in the form of a lockable knee joint.

## Revendications

1. Dispositif d'articulation orthopédique comprenant une partie supérieure (1) et une partie inférieure (2) montée sur celle-ci de manière à pouvoir pivoter autour d'un axe de pivotement (3), un dispositif d'amortissement hydraulique (4) étant disposé entre la partie supérieure (1) et la partie inférieure (2), qui comprend un piston (7) monté dans un cylindre (6) et pourvu d'une tige de piston (5),
**caractérisé en ce que**
le piston (7) est monté solidairement en rotation dans le cylindre (6), et
un insert (72) réglable par l'intermédiaire de la tige de piston (5) est disposé dans le piston (7) avec une fente d'étranglement (73) variable.

2. Dispositif d'articulation orthopédique selon la revendication 1, **caractérisé en ce que** l'insert (72) est mobile en translation dans la direction de déplacement du piston (7) ou est de forme ronde et est monté de façon mobile en rotation dans le piston (7).

3. Dispositif d'articulation orthopédique selon la revendication 1 ou 2, **caractérisé en ce que** l'insert (72) est monté dans une bague de réglage (71) montée solidairement en rotation dans le piston (7).

4. Dispositif d'articulation orthopédique selon la revendication 3, **caractérisé en ce qu'**un canal respectif (75, 76) est formé dans la bague de réglage (71) et dans l'insert (72), qui est en liaison fluidique avec la fente d'étranglement (73) formée entre la bague de réglage (71) et l'insert (72).

5. Dispositif d'articulation orthopédique selon l'une des revendications 1 à 4,
**caractérisé en ce que** la fente d'étranglement (73) présente une section transversale variable sur un trajet de réglage de l'insert (72).

6. Dispositif d'articulation orthopédique selon l'une des revendications 1 à 5,
**caractérisé en ce que** le piston (6) et le cylindre (7) sont de forme ovale.

7. Dispositif d'articulation orthopédique selon l'une des revendications 1 à 6,
**caractérisé en ce qu'**au moins un clapet anti-retour (77, 78) est disposé dans le piston (7), lequel ferme une dérivation (76, 79) de la fente d'étranglement (73).

8. Dispositif d'articulation orthopédique selon la revendication 7, **caractérisé en ce qu'**au moins un clapet anti-retour (77, 78) est sollicité par un ressort.

9. Dispositif d'articulation orthopédique selon l'une des revendications 1 à 8,
**caractérisé en ce que** le piston (7) et/ou le cylindre (6) sont fabriqués au moins partiellement en une matière plastique.

10. Dispositif d'articulation orthopédique selon l'une des revendications précédentes,
**caractérisé en ce que** le dispositif d'articulation est réalisé sous forme d'articulation de genou à verrouillage.
